# EUROPEAN PATENT APPLICATION

(11) **EP 1 790 631 A1**
(43) Date of publication of application: **30.05.2007**
(21) Application number: 04773401.7
(22) Date of filing: 17.09.2004
(51) Int. Cl.: C07C 211/61, C09K 11/06, H05B 33/14

(54) **AROMATIC AMINE DERIVATIVE AND ORGANIC ELECTROLUMINESCENT DEVICE USING SAME**

(71) Applicant: IDEMITSU KOSAN CO., LTD., Tokyo 100-8321 (JP)
(72) Inventor: FUNAHASHI, Masakazu, 2990293 (JP)
(74) Representative: Gille Hrabal Struck Neidlein Prop Roos
(86) International application number: PCT/JP2004/014017
(87) International publication number: WO 2006/030527

(57) **Abstract**

An aromatic amine derivative having a specific structure in which diphenylamino groups having substituents are bonded with the unsubstituted pyrene structure, and an organic electroluminescence device comprising an organic thin film layer which comprises one layer or a plurality of layers comprising at least a light emitting layer and is disposed between an anode and a cathode, wherein at least one layer in the organic thin film layer comprises the aromatic amine derivative described above singly or as a component of a mixture. The organic electroluminescence device has a long life and emits blue light with a great efficiency. The aromatic amine derivative enables to obtain the device.

## Description

### TECHNICAL FIELD

The present invention relates to an organic electroluminescent device which is used as the light source such as a planar light emitting member of wall televisions and a back light of displays, has a long life and exhibits a great efficiency of light emission and a novel aromatic amine derivative enabling to obtain the device.

### BACKGROUND ART

Organic electroluminescence (hereinafter, "electroluminescence" and "electroluminescent" will be referred to as "EL") devices using organic substances are expected to be useful as the inexpensive full color display device of the solid light emission type having a great area, and various developments have been made. In general, an EL device is constituted with a light emitting layer and a pair of electrodes disposed at both sides of the light emitting layer. For the light emission, electrons are injected at the side of the cathode, and holes are injected at the side of the anode when an electric field is applied. The electrons are combined with the holes in the light emitting layer to form excited states, and the energy formed when the excited states returns to the ground state is discharged as light.

Conventional organic EL devices require greater driving voltages and exhibit smaller luminances of emitted light and smaller efficiencies of light emission than those of inorganic light emitting diodes. Moreover, marked deterioration in the properties takes place, and the devices have not been used in practical applications. The properties of the organic EL devices are being improved gradually, but a greater efficiency of light emission and a longer life are required.

For example, a technology in which a single anthracene compound is used as the organic light emitting material is disclosed (Japanese Patent Application Laid-Open No. Heisei 11(1999)-3782). However, since the luminance is as small as 1650 cd/m² at a current density of 165 mA/cm², and the efficiency is as small as 1 cd/A in accordance with this technology, this technology cannot be used in the practical applications. A technology in which a single bisanthracene compound is used as the organic light emitting material is disclosed (Japanese Patent Application Laid-Open No. Heisei 8(1996)-12600). However, the efficiency is as small as about 1 to 3 cd/A in this technology, and improvement is required for the practical application. A technology in which a mono- or bisanthracene compound and a distyryl compound are used for the layer of the organic light emitting medium is disclosed (International Patent Application Laid-Open No. WO 00/06402). However, the obtained device does not have a sufficient life, and further improvement is required.

When an organic EL device is prepared using a diaminopyrene derivative described in Japanese Patent Application Laid-Open No. Heisei 4(1992)-175395 as the doping material, an organic EL device exhibiting a great efficiency of light emission can be obtained. However, this device does not have a sufficiently long life, and further improvement is required. In Japanese Patent Application Laid-Open No. Heisei 10(1998)-251633, a 1,6-substituted diaminopyrene compound is described as an example. When this compound is used as the light emitting material, the molecular weight is great, and the compound tends to be decomposed during vapor deposition.

### DISCLOSURE OF THE INVENTION

The present invention has been made to overcome the above problems and has an object of providing an organic EL device having a long life and exhibiting a great efficiency of light emission and an aromatic amine derivative enabling to obtain the device.

As the result of intensive studies by the present inventors to develop the aromatic amine derivative having the advantageous properties described above and the organic EL device using the derivative, it was found that the object could be achieved by utilizing an aromatic amine derivative represented by general formula (a) shown in the following in which diphenylamino groups having substituents are bonded with the unsubstituted pyrene structure. The present invention has been completed based on the knowledge.

The present invention provides an aromatic amine derivative represented by following general formula (a): 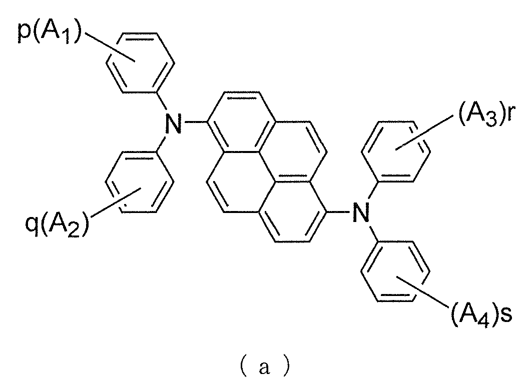 wherein A₁, A₂ and A₃ each independently represent an alkyl group having 1 to 20 carbon atoms, a substituted or unsubstituted aryl group having 5 to 25 carbon atoms, a substituted or unsubstituted aralkyl group having 6 to 25 carbon atoms, a substituted or unsubstituted cycloalkyl group having 3 to 25 carbon atoms, a substituted or unsubstituted alkoxyl group having 1 to 20 carbon atoms, a substituted or unsubstituted aryloxyl group having 5 to 25 carbon atoms, a substituted or unsubstituted arylamino group having 5 to 25 carbon atoms or a substituted or unsubstituted alkylamino group having 1 to 20 carbon atoms;
A₄ represents an alkyl group having 2 to 20 carbon atoms, a substituted or unsubstituted aryl group having 5 to 25 carbon atoms, a substituted or unsubstituted cycloalkyl group having 3 to 25 carbon atoms, a substituted or unsubstituted alkoxyl group having 1 to 20 carbon atoms, a substituted or unsubstituted aryloxyl group having 5 to 25 carbon atoms, a substituted or unsubstituted arylamino group having 5 to 25 carbon atoms or a substituted or unsubstituted alkylamino group having 1 to 20 carbon atoms;
cases in which substituents to any of groups represented by A₁, A₂, A₃ and A₄ are groups having vinyl group are excluded;
p, q, r and s each represent an integer of 0 to 5, and p+q+r+s≧1; when p represents an integer of 2 or greater, a plurality of groups represented by A₁ may be same with or different from each other and may be bonded to each other to form a saturated or unsaturated ring; when q represents an integer of 2 or greater, a plurality of groups represented by A₂ may be same with or different from each other and may be bonded to each other to form a saturated or unsaturated ring; when r represents an integer of 2 or greater, a plurality of groups represented by A₃ may be same with or different from each other and may be bonded to each other to form a saturated or unsaturated ring; and when s represents an integer of 2 or greater, a plurality of groups represented by A₄ may be same with or different from each other and may be bonded to each other to form a saturated or unsaturated ring.

The present invention also provides an organic electroluminescence device comprising an anode, a cathode and an organic thin film layer which comprises a single layer or a plurality of layers comprising at least a light emitting layer and is disposed between the anode and the cathode, wherein at least one layer in the organic thin film layer comprises the aromatic amine derivative described above singly or as a component of a mixture.

### BRIEF DESCRIPTION OF THE DRAWINGS

Figure 1 shows a diagram exhibiting the NMR spectrum of Compound (1) which is the aromatic amine derivative of the present invention.
Figure 2 shows a diagram exhibiting the NMR spectrum of Compound (2) which is the aromatic amine derivative of the present invention.
Figure 3 shows a diagram exhibiting the NMR spectrum of Compound (5) which is the aromatic amine derivative of the present invention.
Figure 4 shows a diagram exhibiting the NMR spectrum of Compound (6) which is the aromatic amine derivative of the present invention.
Figure 5 shows a diagram exhibiting the NMR spectrum of Compound (13) which is the aromatic amine derivative of the present invention.
Figure 6 shows a diagram exhibiting the NMR spectrum of Compound (15) which is the aromatic amine derivative of the present invention.
Figure 7 shows a diagram exhibiting the NMR spectrum of Compound (20) which is the aromatic amine derivative of the present invention.
Figure 8 shows a diagram exhibiting the NMR spectrum of Compound (26) which is the aromatic amine derivative of the present invention.

### THE MOST PREFERRED EMBODIMENT TO CARRY OUT THE INVENTION

The aromatic amine derivative of the present invention comprises an aromatic amine derivative represented by the above general formula (a).

In general formula (a), A₁, A₂ and A₃ each independently represent an alkyl group having 1 to 20 carbon atoms (preferably 1 to 6 carbon atoms), a substituted or unsubstituted aryl group having 5 to 25 carbon atoms (preferably 5 to 10 carbon atoms), a substituted or unsubstituted aralkyl group having 6 to 25 carbon atoms (preferably 6 to 10 carbon atoms), a substituted or unsubstituted cycloalkyl group having 3 to 25 carbon atoms (preferably 3 to 10 carbon atoms), a substituted or unsubstituted alkoxyl group having 1 to 20 carbon atoms (preferably 1 to 6 carbon atoms), a substituted or unsubstituted aryloxyl group having 5 to 25 carbon atoms (preferably 5 to 10 carbon atoms), a substituted or unsubstituted arylamino group having 5 to 25 carbon atoms (preferably 5 to 10 carbon atoms) or a substituted or unsubstituted alkylamino group having 1 to 20 carbon atoms (preferably 1 to 6 carbon atoms).

Examples of the alkyl group represented by A₁, A₂ and A₃ include methyl group, ethyl group, propyl group, isopropyl group, butyl group, sec-butyl group, tert-butyl group, pentyl group, hexyl group, heptyl group, octyl group, stearyl group, 2-phenylisopropyl group, trichloromethyl group, trifluoromethyl group, benzyl group, α-phenoxybenzyl group, α,α-dimethylbenzyl group, α,α-methylphenylbenzyl group, α,α-ditrifluoromethylbenzyl group, triphenylmethyl group and α-benzyloxybenzyl group. Among these groups, methyl group, ethyl group, isopropyl group, butyl group, see-butyl group and tert-butyl group are preferable.

Examples of the aryl group represented by A₁, A₂ and A₃ include phenyl group, 2-methylphenyl group, 3-methylphenyl group, 4-methylphenyl group, 4-ethylphenyl group biphenyl group, 4-methylbiphenyl group, 4-ethylbiphenyl group, 4-cyclohexylbiphenyl group, terphenyl group, 3,5-dichlorophenyl group, naphthyl group, 5-methylnaphthyl group, anthryl group and pyrenyl group.

Examples of the aralkyl group represented by A₁, A₂ and A₃ include benzyl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, 2-β-naphthylisopropyl group, 1-pyrrolylmethyl group, 2-(1-pyrrolyl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group, o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyanobenzyl group, 1-hydroxy-2-phenylisopropyl group and 1-chloro-2-phenylisopropyl group.

Examples of the cycloalkyl group represented by A₁, A₂ and A₃ include cyclopropyl group, cyclobutyl group, cyclopentyl group, cyclohexyl group, norbornene group and adamantyl group.

Examples of the alkoxyl group represented by A₁, A₂ and A₃ include methoxyl group, ethoxyl group, propoxyl group, isopropoxyl group, butoxyl group, isobutoxyl group, sec-butoxyl group, tert-butoxyl group, various types of pentyloxyl groups and various types of hexyloxyl groups.

Examples of the aryloxyl group represented by A₁, A₂ and A₃ include phenoxyl group, tolyloxyl group and naphthyloxyl group.

Examples of the arylamino group represented by A₁, A₂ and A₃ include diphenylamino group, ditolylamino group, isopropyldiphenylamiono group, t-butyldiphenylamino group, diisopropyldiphenylamino group, di-t-butyldiphenylamino group, dinaphthylamino group and naphthylphenylamino group.

Examples of the alkylamino group represented by A₁, A₂ and A₃ include dimethylamino group, diethylamino group and dihexylamino group.

In general formula (a), A₄ represents an alkyl group having 2 to 20 carbon atoms (preferably 2 to 6 carbon atoms), a substituted or unsubstituted aryl group having 5 to 25 carbon atoms (preferably 5 to 10 carbon atoms), a substituted or unsubstituted cycloalkyl group having 3 to 25 carbon atoms (preferably 3 to 10 carbon atoms), a substituted or unsubstituted alkoxyl group having 1 to 20 carbon atoms (preferably 1 to 6 carbon atoms), a substituted or unsubstituted aryloxyl group having 5 to 25 carbon atoms (preferably 5 to 10 carbon atoms), a substituted or unsubstituted arylamino group having 5 to 25 carbon atoms (preferably 5 to 10 carbon atoms) or a substituted or unsubstituted alkylamino group having 1 to 20 carbon atoms (preferably 1 to 6 carbon atoms).

Examples of the alkyl group represented by A₄ include ethyl group, propyl group, isopropyl group, butyl group, sec-butyl group, tert-butyl group, pentyl group, hexyl group, heptyl group, octyl group, stearyl group, 2-phenylisopropyl group, trichloromethyl group, trifluoromethyl group, benzyl group, α-phenoxybenzyl group, α,α-dimethylbenzyl group, α,α-methylphenylbenzyl group, α,αditrifluoromethylbenzyl group, triphenylmethyl group and α-benzyloxybenzyl group. Among these groups, ethyl group, isopropyl group, butyl group, sec-butyl group and tert-butyl group are preferable.

Examples of the aryl group, the cycloalkyl group, the alkoxyl group, the aryloxyl group, the arylamino group and the alkylamino group represented by A₄ include the groups described above as the examples of the corresponding groups represented by A₁, A₂, A₃ and A₄.

However, cases in which substituents to any of groups represented by A₁, A₂, A₃ and A₄ are groups having vinyl group are excluded.

In general formula (a) in the present invention, it is preferable that at least one of A₁, A₂, A₃ and A₄ represents a branched alkyl group such as isopropyl group, sec-butyl group and tert-butyl group and more preferably isopropyl group or tert-butyl group.

It is also preferable that at least one of A₁, A₂, A₃ and A₄ represents methyl group.

**p, q, r** and **s** each represent an integer of 0 to 5, and p+q+r+s≧1. When **p** represents an integer of 2 or greater, a plurality of groups represented by A₁ may be same with or different from each other and may be bonded to each other to form a saturated or unsaturated ring. When q represents an integer of 2 or greater, a plurality of groups represented by A₂ may be same with or different from each other and may be bonded to each other to form a saturated or unsaturated ring. When r represents an integer of 2 or greater, a plurality of groups represented by A₃ may be same with or different from each other and may be bonded to each other to form a saturated or unsaturated ring. When s represents an integer of 2 or greater, a plurality of groups represented by A₄ may be same with or different from each other and may be bonded to each other to form a saturated or unsaturated ring.

Examples of the saturated or unsaturated ring include cyclic structures of the groups described above as the examples of the aryl group and the cycloalkyl groups.

In general formula (a) in the present invention, it is preferable that at least one of p, q, r and s represents an integer of 2 or greater.

Examples of the aromatic amine derivative represented by general formula (a) in the present invention are shown in the following. However, the aromatic amine derivative is not limited to the compounds shown as the examples. Me in the following formulae represents methyl group.

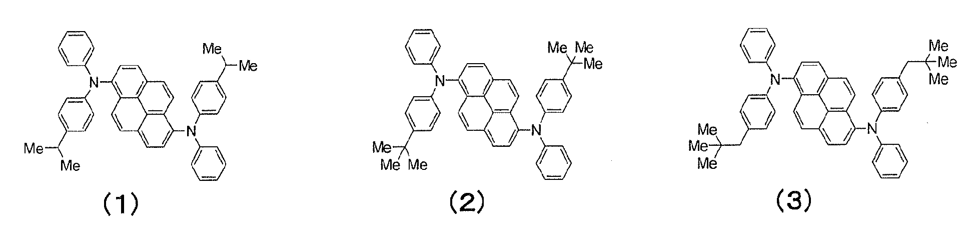

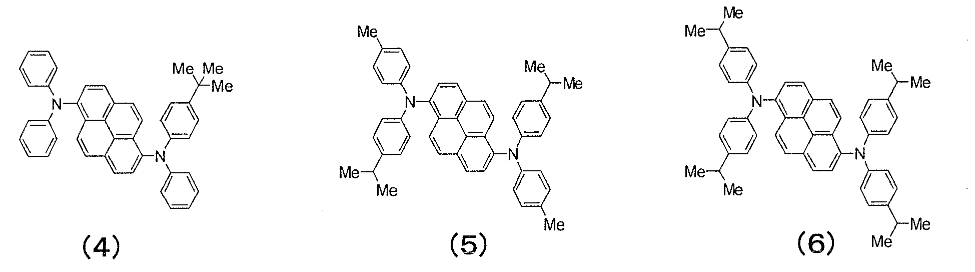

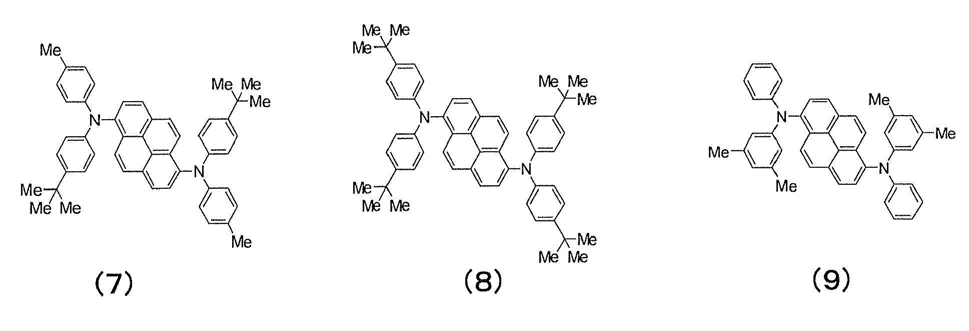

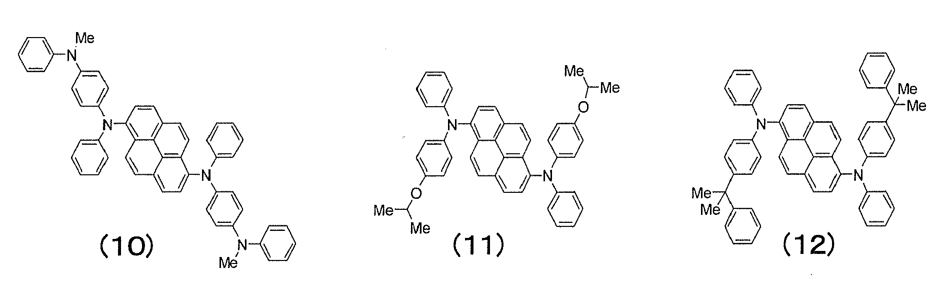

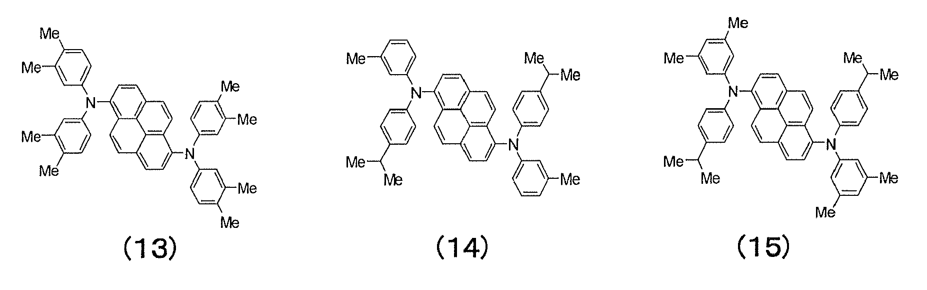

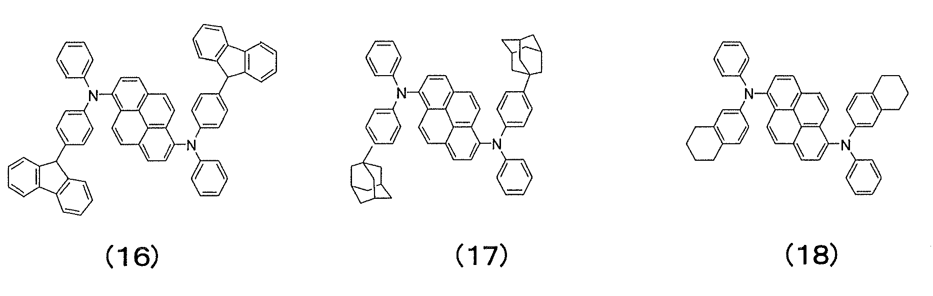

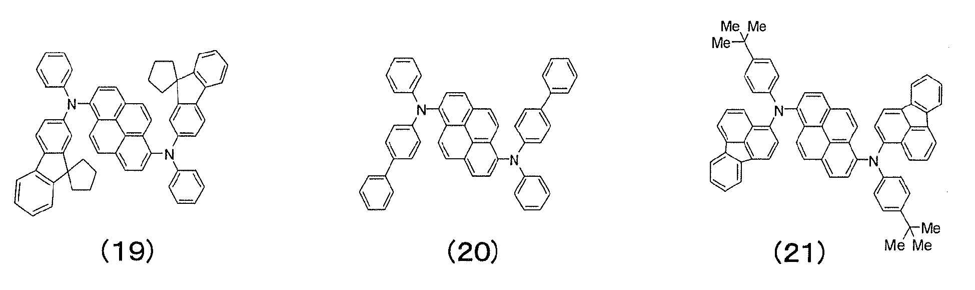

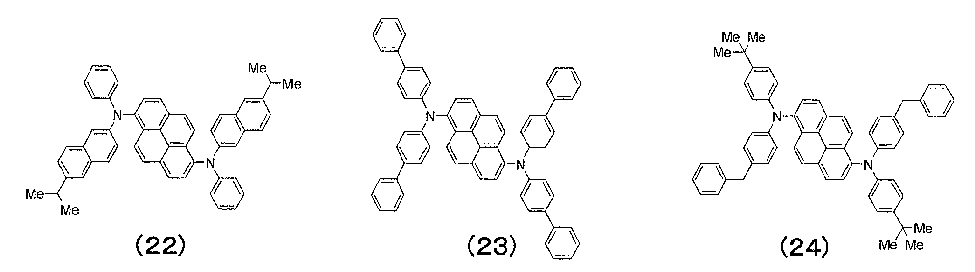

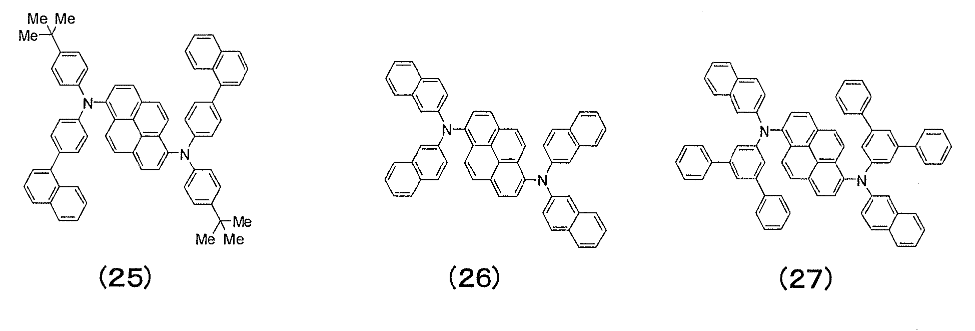

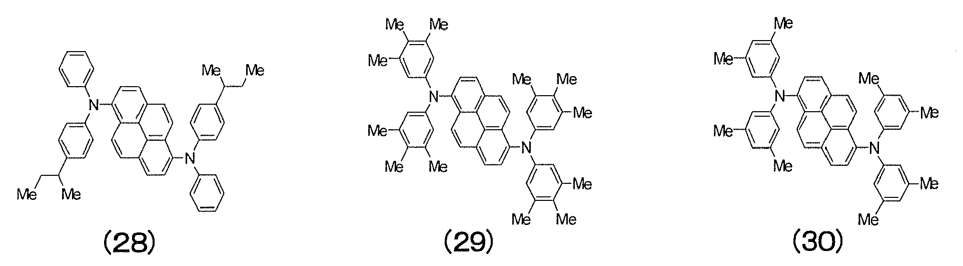

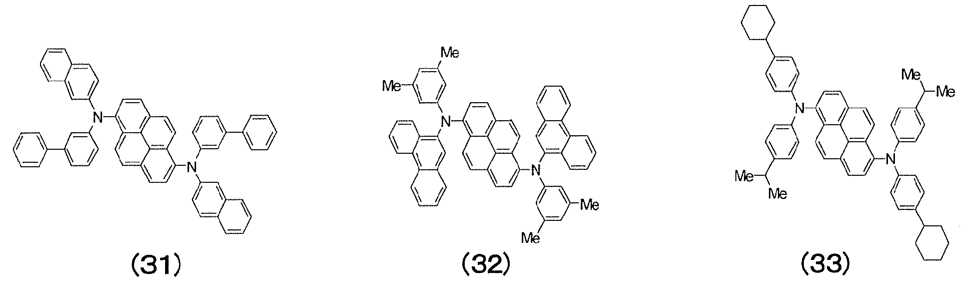

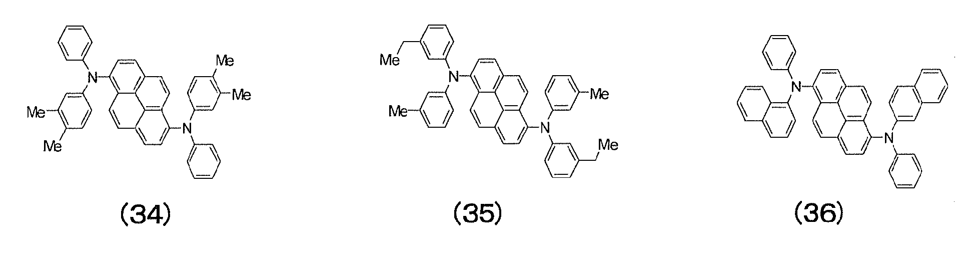

As the aromatic amine derivative of the present invention, Compounds (1), (2), (5), (6), (7), (8), (9), (13), (15), (20), (26), (29), (30) and (33) are preferable among the above compounds.

The aromatic amine derivative of the present invention represented by general formula (a) provides an increased life due to suppressed association between the compounds since diphenylamino groups having substituents are bonded with the pyrene structure and, in particular, the substituent is an alkyl group which is preferably an alkyl group having two or more substituents or a branched alkyl group. The aromatic amine derivative exhibits a strong fluorescent property in the solid state, an excellent light emitting property under an electric field and a quantum efficiency of fluorescence of 0.3 or greater. Moreover, since excellent properties of injecting and transporting holes from the metal electrode or the organic thin film layer and excellent properties of injecting and transporting electrons from the metal electrode or the organic thin film layer are exhibited simultaneously, the aromatic amine derivative is effectively used as the light emitting material and, in particular, as the doping material for organic EL devices. Other hole transporting materials, electron transporting materials and doping materials may be used in combination.

The organic EL device of the present invention is an organic EL device comprising an anode, a cathode and an organic thin film layer which comprises a single layer or a plurality of layers comprising at least a light emitting layer and is disposed between the anode and the cathode. When the organic thin film layer has a single layer, a light emitting layer is disposed between the anode and the cathode. The light emitting layer comprises a light emitting material and may further comprise a hole injecting material or an electron injecting material for transporting holes injected from the anode or electrons injected from the cathode, respectively, to the light emitting material. The aromatic amine derivative represented by general formula (a) exhibits the excellent light emitting property and the excellent hole injecting property, hole transporting property, electron injecting property and electron transporting property, the derivative can be used for the light emitting layer as the light emitting material or the doping material.

In the organic EL device of the present invention, it is preferable that the light emitting layer comprises the aromatic amine derivative of the present invention in an amount of 0.1 to 20% by weight and more preferably in an amount of 1 to 10% by weight. Since the aromatic amine derivative of the present invention represented by general formula (a) exhibits the combination of the excellent quantum efficiency of light emission, hole transporting ability and electron transporting ability and forms a uniform thin film, it is possible that the light emitting layer is formed with the aromatic amine derivative alone.

It is preferable that the organic EL device of the present invention, which is a device comprising an anode, a cathode and an organic thin film layer which comprises a plurality of layers comprising at least a light emitting layer and is disposed between the anode and the cathode, comprises an organic layer which comprises the aromatic amine derivative represented by general formula (a) as the main component and is disposed between the anode and the light emitting layer. Examples of the organic layer include the hole injecting layer and the hole transporting layer.

Examples of the organic EL device in which the organic thin film layer has a plurality of layers include organic EL devices having multi-layer laminate structures of (an anode / a hole injecting layer / a light emitting layer / a cathode), (an anode / a light emitting layer / an electron injecting layer / a cathode) and (an anode / a hole injecting layer / a light emitting layer / an electron injecting layer / a cathode).

Conventional light emitting materials, doping materials, hole injecting materials and electron injecting materials may be used for the light emitting layer, where necessary, in combination with the aromatic amine derivative of the present invention. Decreases in the luminance and the life due to quenching can be prevented by using a multi-layer structure for the organic EL device. Where necessary, the light emitting materials, the doping materials, the hole injecting materials and the electron injecting materials may be used in combination. By the use of the doping material, the luminance of the emitted light and the efficiency of the light emission can be increased, and red light or blue light can be emitted. The hole injecting layer, the light emitting layer and the electron injecting layer may each have a structure having two or more layers. In this case, the layer into which holes are injected from the electrode is called the hole injecting layer, and the layer which receives the holes from the hole injecting layer and transports the holes to the light emitting layer is called the hole transporting layer. Similarly, the layer into which electrons are injected from the electrode is called the electron injecting layer, and the layer which receives the electrons from the electron injecting layer and transports the electrons to the light emitting layer is called the electron transporting layer. These layers are selected and used in accordance with various factors such as the energy level of the material, the heat resistance of the material and the adhesion of the material with the organic layer or the metal electrode.

Examples of the light emitting material and the doping material which can be used for the light emitting layer in combination with the aromatic amine derivative of the present invention include anthracene, naphthalene, phenanthrene, pyrene, tetracene, coronene, chrysene, fluorescein, perylene, phthaloperylene, naphthaloperylene, perynone, phthaloperynone, naphthaloperynone, diphenylbutadiene, tetraphenylbutadiene, coumarine, oxadiazole, aldazine, bisbenzoxazoline, bisstyryl, pyrazine, cyclopentadiene, metal complex compounds of quinoline, metal complex compounds of aminoquinoline, metal complex compounds of benzoquinoline, imine, diphenylethylene, vinylanthracene, diaminocarbazole, pyran, thiopyrane, polymethine, melocyanine, oxinoid compounds chelated with imidazole, quinacridone, rubrene and fluorescent coloring agents. However, the light emitting material and the doping material are not limited to the compounds described above.

As the hole injecting material, compounds which have the ability of transporting holes, exhibit the excellent effect of injection of holes from the anode and the excellent effect of injection of holes to the light emitting layer or the light emitting material, prevent transfer of excimers formed in the light emitting layer to the electron injecting layer or the electron injecting material and have excellent ability of forming a thin film are preferable. Examples of the hole injecting material include phthalocyanine derivatives, naphthalocyanine derivatives, porphyrin derivatives, oxazoles, oxadiazoles, triazoles, imidazoles, imidazolones, imidazolethiones, pyrazolines, pyrazolones, tetrahydroimidazoles, hydrazones, acylhydrazones, polyarylalkanes, stilbenes, butadienes, triphenylamines of the benzidine type, triphenyl- amines of the styrylamine type, triphenylamines of the diamine type, derivatives of these compounds and macromolecular materials such as polyvinylcarbazole, polysilanes and electrically conductive macromolecules. However, the hole injecting material is not limited to the compounds described above.

Among the hole injecting materials which can be used in the organic EL device of the present invention, aromatic tertiary amine derivatives and phthalocyanine derivatives are more effective hole injecting materials.

Examples of the aromatic tertiary amine derivative include triphenylamine, tritolylamine, tolyldiphenylamine, N,N'-diphenyl-N,N'-(3-methylphenyl)-1,1'-biphenyl-4,4'-diamine, N,N,N',N'-(4-methylphenyl)-1,1'-phenyl-4,4'-diamine, N,N,N',N'-(4-methylphenyl)-1,1'-biphenyl-4,4'-diamine, N,N'-diphenyl-N,N'-dinaphthyl-1,1'-biphenyl-4,4'-diamine, N,N'-(methylphenyl)-N,N'-(4-n-butylphenyl)phenanthrene-9,10-diamine, N,N-bis(4-di-4-tolylaminophenyl)-4-phenylcyclohexane and oligomer and polymers having the skeleton structure of the above aromatic tertiary amines. However, the aromatic tertiary amine derivative is not limited to the compounds described above.

Examples of the phthalocyanine (Pc) derivative include phthalocyanine derivatives and naphthalocyanine derivatives such as H₂Pc, CuPc, CoPc, NiPc, ZnPc, PdPc, FePc, MnPc, ClAlPc, ClGaPc, ClInPc, ClSnPc, Cl₂SiPc, (HO)AlPc, (HO)GaPc, VOPc, TiOPc, MoOPc, GaPc-O-GaPc. However, the phthalocyanine derivative is not limited to the compounds described above.

In the organic EL device of the present invention, it is preferable that a layer comprising the aromatic amine derivative and/or the phthalocyanine derivative described above such as the hole transporting layer or the hole injecting layer described above is disposed between the light emitting layer and the anode.

As the electron injecting material, compounds which have the ability of transporting electrons, exhibit the effect of injection of electrons from the cathode and the excellent effect of injection of electrons into the light emitting layer or the light emitting material, prevent transfer of excimers formed in the light emitting layer into the hole injecting layer and have excellent ability of forming a thin film, are preferable. Examples of the electron injecting material include fluorenone, anthraquinodimethane, diphenoquinones, thiopyrane dioxides, oxazoles, oxadiazoles, triazoles, imidazoles, perylenetetracarboxylic acid, fluorenylidenemethane, anthraquinodimethane, anthrone and derivatives of these compounds. However, the electron injecting material is not limited to the compounds described above. The property of charge injection can also be improved by adding an electron-accepting substance to the hole injecting material and an electron-donating substance to the electron injecting material.

In the organic EL device of the present invention, more effective electron injecting transporting materials are metal complex compounds and five-membered cyclic derivatives having nitrogen atom.

Examples of the metal complex compound include 8-hydroxyquinolinatolithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)-beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum and bis(2-methyl-8-quinolinato)(2-naphtholato)gallium. However, the metal complex compound is not limited to the compounds described above.

As the five-membered cyclic derivative having nitrogen atom, for example, derivatives of oxazole, thiazole, oxadiazole, thiadiazole and triazole are preferable. Examples of the five-membered cyclic derivative having nitrogen atom include 2,5-bis(1-phenyl)-1,3,4-oxazole, dimethylPOPOP, 2,5-bis(1-phenyl)-1,3,4-thiazole, 2,5-bis(1-phenyl)-1,3,4-oxadiazole, 2-(4'-tert-butylphenyl)-5-(4"-biphenyl)-1,3,4-oxadiazole, 2,5-bis(1-naphthyl)-1,3,4-oxadiazole, 1,4-bis[2-(5-phenyloxadiazolyl)]benzene, 1,4-bis[2-(5-phenyloxadiazolyl)-4-tert-butylbenzene], 2-(4'-tert-butylphenyl)-5-(4"-biphenyl)-1,3,4-thiadiazole, 2,5-bis(1-naphthyl)-1,3,4-thiazole, 1,4-bis-[2-(5-phenylthiadiazolyl)]benzene, 2-(4'-tert-butylphenyl)-5-(4"-biphenyl)-1,3,4-triazole, 2,5-bis(1-naphthyl)-1,3,4-triazole and 1,4-bis[2-(5-phenyltriazolyl)]benzene. However, the five-membered cyclic derivative having nitrogen atom is not limited to the compounds described above.

In the organic EL device of the present invention, the light emitting layer may further comprise at least one of light emitting materials, doping materials, hole injecting materials and electron injecting materials in the same layer in addition to the aromatic amine derivative represented by general formula (a). A protective layer may be formed on the surface of the device or the entire device may be protected with a silicone oil or a resin so that stability of the organic EL device obtained in accordance with the present invention with respect to the temperature, the moisture and the atmosphere is improved.

As the electrically conductive material used for the anode in the organic EL device of the present invention, a material having a work function greater than 4 eV is suitable. Carbon, aluminum, vanadium, iron, cobalt, nickel, tungsten, silver, gold, platinum, palladium, alloys of these metals, metal oxides such as tin oxide and indium oxide used for ITO substrates and NESA substrates, and organic electrically conductive resins such as polythiophene and polypyrrol can be used. As the electrically conductive material used for the cathode, a material having a work function smaller than 4 eV is suitable. Magnesium, calcium, tin, lead, titanium, yttrium, lithium, ruthenium, manganese, aluminum, lithium fluoride and alloys of these metals can be used, but the material is not limited to these materials. Typical examples of the alloy include magnesium/silver, magnesium/indium and lithium/aluminum, but the alloy is not limited to these alloys. The ratio of the amounts of the components in the alloy is controlled by the temperature of the sources of vapor deposition, the atmosphere and the degree of vacuum and adjusted to a suitable value. The anode and the cathode may have a structure having two or more layers, where necessary.

In the organic EL device of the present invention, it is desirable that at least one face of the device is sufficiently transparent in the wavelength range of the light emitted from the device so that the emitted light is efficiently obtained. It is desirable that the substrate is transparent. The transparent electrode is disposed in accordance with a process such as the vapor deposition process or the sputtering process using the above electrically conductive material in a manner such that the prescribed transparency is assured. It is preferable that the electrode on the light emitting face has a transmittance of light of 10% or greater. The substrate is not particularly limited as long as the substrate has sufficient mechanical and thermal strength and transparency. Examples of the substrate include glass substrates and transparent resin films. Examples of the transparent resin films include films of polyethylene, ethylene-vinyl acetate copolymers, ethylene-vinyl alcohol copolymers, polypropylene, polystyrene, polymethyl methacrylate, polyvinyl chloride, polyvinyl alcohol, polyvinyl butyral, nylons, polyether ether ketones, polysulfones, polyether sulfones, tetrafluoroethylene-perfluoroalkyl vinyl ether copolymers, polyvinyl fluoride, tetrafluoroethylene-ethylene copolymers, tetrafluoroethylene-hexafluoropropylene copolymers, polychlorotrifluoroethylene, polyvinylidene fluoride, polyesters, polycarbonates, polyurethanes, polyimides, polyether imides, polyimides and polypropylene.

To the formation of the layers in the organic EL device of the present invention, any of dry film forming processes such as the vacuum vapor deposition process, the sputtering process, the plasma process and the ion plating process and wet film forming processes such as the spin coating process, the dipping process and the flow coating process can be applied. It is necessary that the thickness of the film is set at a suitable value although the thickness of the layer is not particularly limited. When the thickness is excessively great, a great voltage must be applied to obtain the prescribed output, and the efficiency decreases. When the thickness is excessively small, defects such as pin holes are formed, and the sufficient luminance of the emitted light cannot be obtained under application of an electric field. It is suitable that the thickness is in the range of 5 nm to 10 µm, and a thickness in the range of 10 nm to 0.2 µm is preferable.

In the wet process, the materials forming each layer are dissolved or dispersed in a suitable solvent such as ethanol, chloroform, tetrahydrofuran and dioxane, and a thin film is formed from the solution or the dispersion. Any of the above solvents can be used. Suitable resins and additives may be used in any of the organic thin films to improve the property for film formation and prevent formation of pin holes. Examples of the resin which can be used include insulating resins such as polystyrene, polycarbonates, polyarylates, polyesters, polyamides, polyurethanes, polysulfones, polymethyl methacrylate, polymethyl acrylate and cellulose, copolymers of the insulating resins, photoconductive resins such as poly-N-vinylcarbazoles and polysilanes and electrically conductive resins such as polythiophene and polypyrrol. Examples of the additive include antioxidants, ultraviolet light absorbents and plasticizers.

As described above, the organic EL device having a long life and exhibiting an excellent efficiency of light emission can be obtained by using the aromatic amine derivative of the present invention for the organic thin film layer in the organic EL device.

The organic EL device of the present invention can be utilized as a planar light emitting body such as flat panel displays of wall televisions, a back light for copiers, printers and liquid crystal displays, a light source for instruments, a display plate and a marking light. The material of the present invention can also be used in the fields other than the organic EL device such as electronic photo-sensitive materials, photo-electric converters, solar batteries and image sensors in addition.

### EXAMPLES

The present invention will be described more specifically with reference to examples in the following. However, the present invention is not limited to the examples.

### Synthesis Example 1 Synthesis of Compound (1)

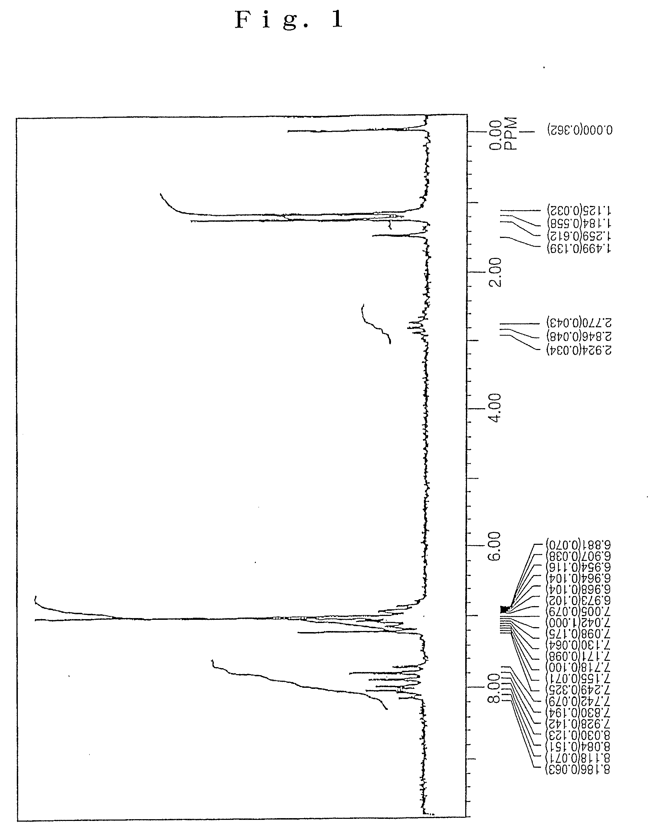

Under the stream of argon, 3.6 g (10 mmole) of 1,6-dibromopyrene, 5.2 g (25 mmole) of 4-isopropyldiphenylamine, 0.03 g (1.5% by mole) of palladium acetate, 0.06 g (3% by mole) of tri-t-butylphosphine, 2.4 g (25 mmole) of t-butoxysodium and 100 ml of dry toluene were placed into a 300 ml three-necked flask equipped with a condenser, and the resultant mixture was heated at 100°C under stirring for one night. After the reaction was completed, the formed crystals were separated by filtration and washed with 50 ml of toluene and 100 ml of methanol, and 5.5 g of a light yellow powder substance was obtained. The obtained product was identified to be Compound (1) from the ¹H-NMR spectrum (Figure 1) and by the measurement in accordance with the field desorption mass spectroscopy (FD-MS) (the yield: 89%).

In the ¹H-NMR spectroscopy, the measurement was conducted using CDCl₃ as the solvent and a Fourier transform nuclear magnetic resonance apparatus R-1900 (90 MHz) manufactured by HITACHI SEISAKUSHO Co., Ltd. or DRX-500 (500 MHz) manufactured by BRUCKER Company.

### Preparation Example 2 (Synthesis of Compound (2))

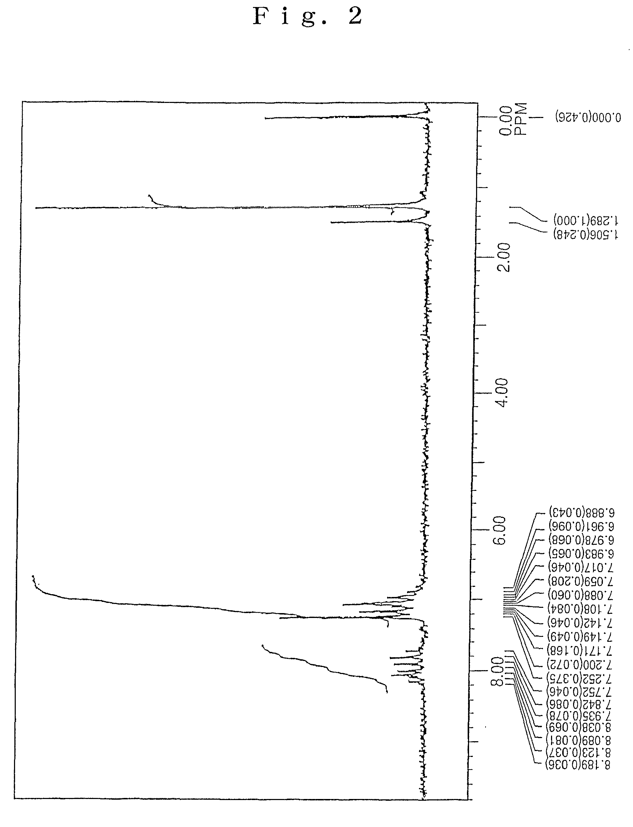

Under the stream of argon, 3.6 g (10 mmole) of 1,6-dibromopyrene, 5.6 g (25 mmole) of 4-t-butyldiphenylamine, 0.03 g (1.5% by mole) of palladium acetate, 0.06 g (3% by mole) of tri-t-butylphosphine, 2.4 g (25 mmole) of t-butoxysodium and 100 ml of dry toluene were placed into a 300 ml three-necked flask equipped with a condenser, and the resultant mixture was heated at 100°C under stirring for one night. After the reaction was completed, the formed crystals were separated by filtration and washed with 50 ml of toluene and 100 ml of methanol, and 5.1 g of a light yellow powder substance was obtained. The obtained product was identified to be Compound (2) from the ¹H-NMR spectrum (Figure 2) and by the measurement in accordance with FD-MS (the yield: 79%). The ¹H-NMR spectrum was obtained under the same condition as that in Synthesis Example 1.

### Preparation Example 3 (Synthesis of Compound (5))

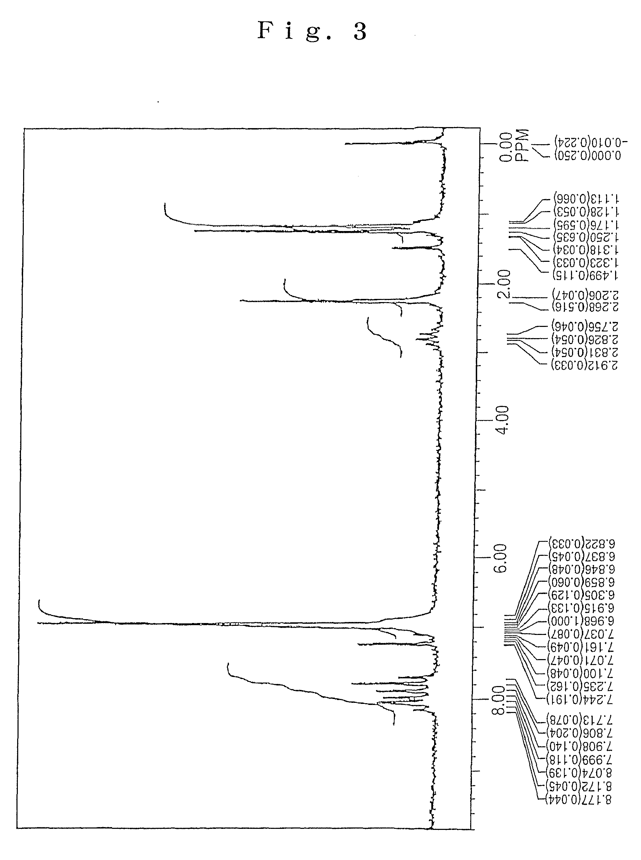

Under the stream of argon, 3.6 g (10 mmole) of 1,6-dibromopyrene, 4.9 g (25 mmole) of 4-isopropylphenyl-p-tolylamine, 0.03 g (1.5% by mole) of palladium acetate, 0.06 g (3% by mole) of tri-t-butylphosphine, 2.4 g (25 mmole) of t-butoxysodium and 100 ml of dry toluene were placed into a 300 ml three-necked flask equipped with a condenser, and the resultant mixture was heated at 100°C under stirring for one night. After the reaction was completed, the formed crystals were separated by filtration and washed with 50 ml of toluene and 100 ml of methanol, and 5.7 g of a light yellow powder substance was obtained. The obtained product was identified to be Compound (5) from the ¹H-NMR spectrum (Figure 3) and by the measurement in accordance with FD-MS (the yield: 93%). The ¹H-NMR spectrum was obtained under the same condition as that in Synthesis Example 1.

### Preparation Example 4 (Synthesis of Compound (6))

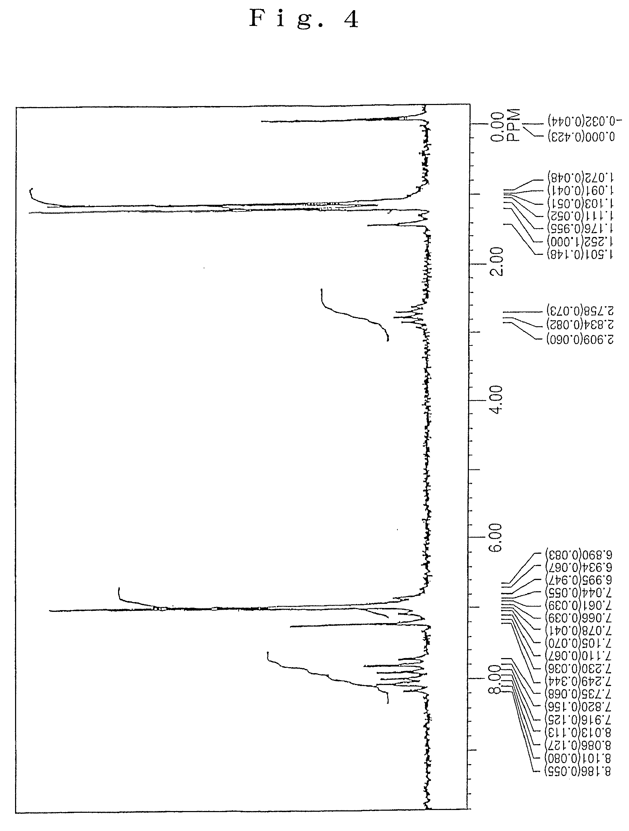

Under the stream of argon, 3.6 g (10 mmole) of 1,6-dibromopyrene, 6.3 g (25 mmole) of bis(4-isopropylphenyl)amine, 0.03 g (1.5% by mole) of palladium acetate, 0.06 g (3% by mole) of tri-t-butylphosphine, 2.4 g (25 mmole) of t-butoxysodium and 100 ml of dry toluene were placed into a 300 ml three-necked flask equipped with a condenser, and the resultant mixture was heated at 100°C under stirring for one night. After the reaction was completed, the formed crystals were separated by filtration and washed with 50 ml of toluene and 100 ml of methanol, and 7.1 g of a light yellow powder substance was obtained. The obtained product was identified to be Compound (6) from the ¹H-NMR spectrum (Figure 4) and by the measurement in accordance with FD-MS (the yield: 97%). The ¹H-NMR spectrum was obtained under the same condition as that in Synthesis Example 1.

### Preparation Example 5 (Synthesis of Compound (13))

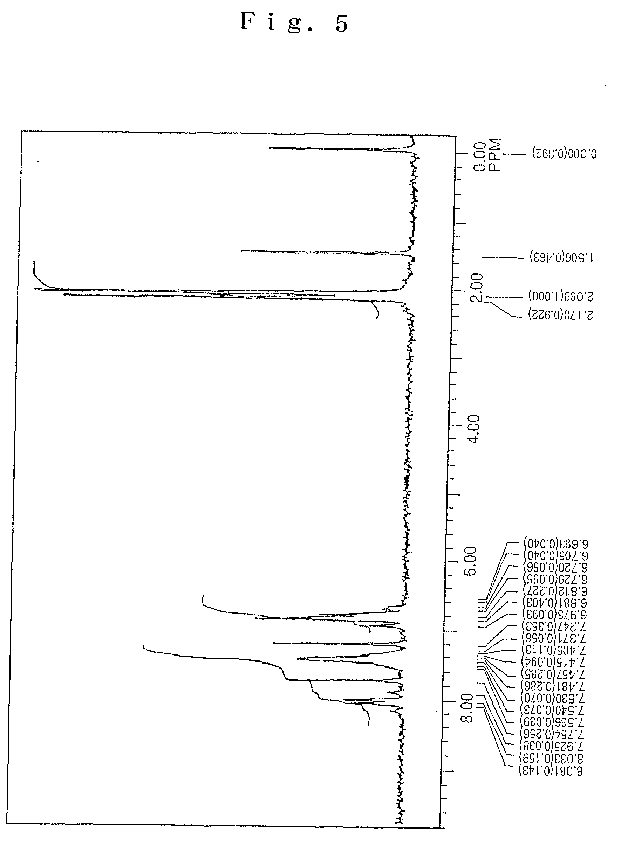

Under the stream of argon, 3.6 g (10 mmole) of 1,6-dibromopyrene, 5.6 g (25 mmole) of bis(3,4-diphenyl)amine, 0.03 g (1.5% by mole) of palladium acetate, 0.06 g (3% by mole) of tri-t-butylphosphine, 2.4 g (25 mmole) of t-butoxysodium and 100 ml of dry toluene were placed into a 300 ml three-necked flask equipped with a condenser, and the resultant mixture was heated at 100°C under stirring for one night. After the reaction was completed, the formed crystals were separated by filtration and washed with 50 ml of toluene and 100 ml of methanol, and 5.9 g of a light yellow powder substance was obtained. The obtained product was identified to be Compound (13) from the ¹H-NMR spectrum (Figure 5) and by the measurement in accordance with FD-MS (the yield: 92%). The ¹H-NMR spectrum was obtained under the same condition as that in Synthesis Example 1.

### Preparation Example 6 (Synthesis of Compound (15))

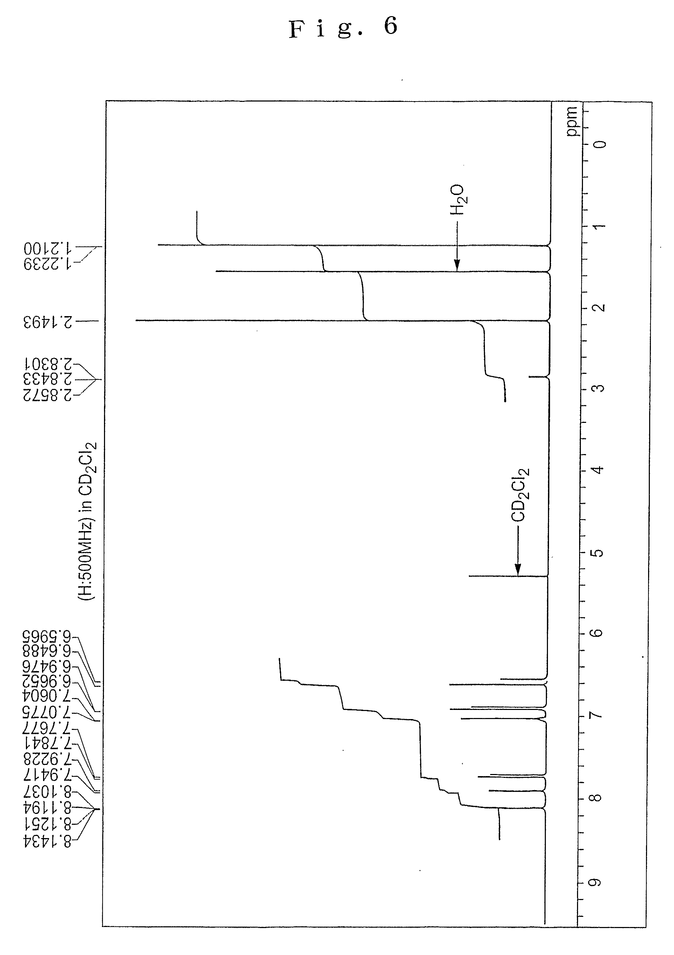

Under the stream of argon, 3.6 g (10 mmole) of 1,6-dibromopyrene, 6.0 g (25 mmole) of 4-isopropylphenyl-3,5-dimethylphenylamine, 0.03 g (1.5% by mole) of palladium acetate, 0.06 g (3% by mole) of tri-t-butylphosphine, 2.4 g (25 mmole) of t-butoxysodium and 100 ml of dry toluene were placed into a 300 ml three-necked flask equipped with a condenser, and the resultant mixture was heated at 100°C under stirring for one night. After the reaction was completed, the formed crystals were separated by filtration and washed with 50 ml of toluene and 100 ml of methanol, and 6.6 g of a light yellow powder substance was obtained. The obtained product was identified to be Compound (15) from the ¹H-NMR spectrum (Figure 6) and by the measurement in accordance with FD-MS (the yield: 98%). The ¹H-NMR spectrum was obtained under the same condition as that in Synthesis Example 1.

### Preparation Example 7 (Synthesis of Compound (20))

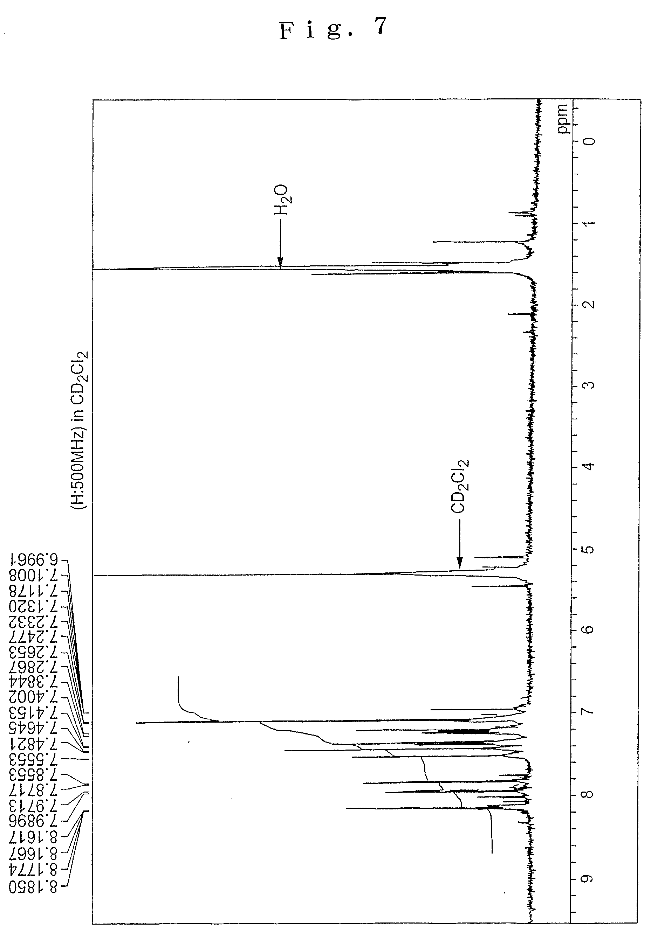

Under the stream of argon, 3.6 g (10 mmole) of 1,6-dibromopyrene, 6.1 g (25 mmole) of 4-phenylbiphenylamine, 0.03 g (1.5% by mole) of palladium acetate, 0.06 g (3% by mole) of tri-t-butylphosphine, 2.4 g (25 mmole) of t-butoxysodium and 100 ml of dry toluene were placed into a 300 ml three-necked flask equipped with a condenser, and the resultant mixture was heated at 100°C under stirring for one night. After the reaction was completed, the formed crystals were separated by filtration and washed with 50 ml of toluene and 100 ml of methanol, and 6.3 g of a light yellow powder substance was obtained. The obtained product was identified to be Compound (20) from the ¹H-NMR spectrum (Figure 7) and by the measurement in accordance with FD-MS (the yield: 92%). The ¹H-NMR spectrum was obtained under the same condition as that in Synthesis Example 1.

### Preparation Example 8 (Synthesis of Compound (26))

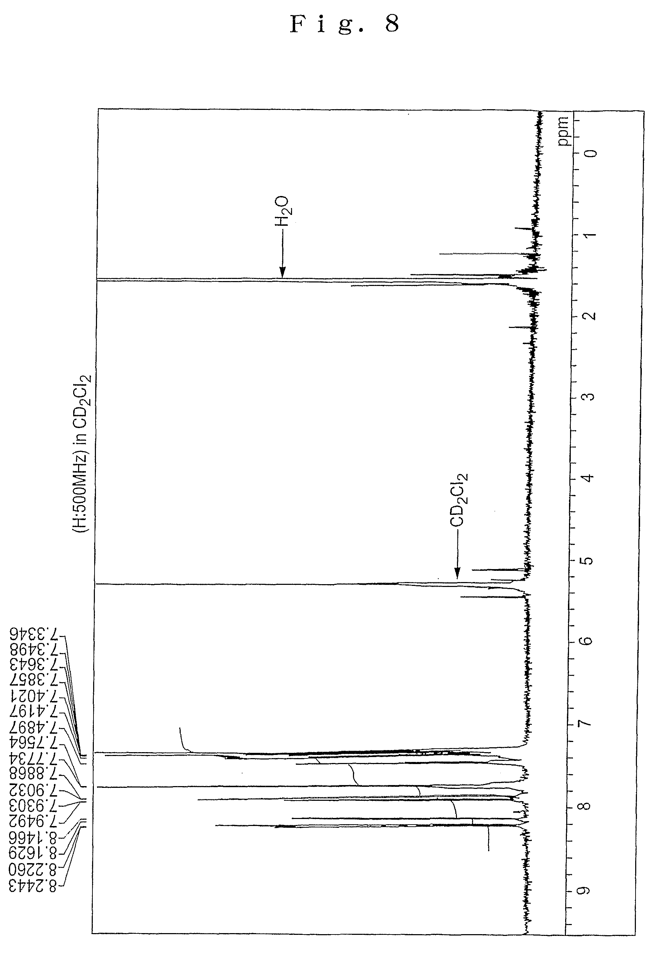

Under the stream of argon, 3.6 g (10 mmole) of 1,6-dibromopyrene, 6.7 g (25 mmole) of di(2-naphthyl)amine, 0.03 g (1.5% by mole) of palladium acetate, 0.06 g (3% by mole) of tri-t-butylphosphine, 2.4 g (25 mmole) of t-butoxysodium and 100 ml of dry toluene were placed into a 300 ml three-necked flask equipped with a condenser, and the resultant mixture was heated at 100°C under stirring for one night. After the reaction was completed, the formed crystals were separated by filtration and washed with 50 ml of toluene and 100 ml of methanol, and 6.6 g of a light yellow powder substance was obtained. The obtained product was identified to be Compound (26) from the ¹H-NMR spectrum (Figure 8) and by the measurement in accordance with FD-MS (the yield: 90%). The ¹H-NMR spectrum was obtained under the same condition as that in Synthesis Example 1.

### Example 1

On a glass substrate having a size of 25 mm × 75 mm × 1.1 mm thickness, a transparent electrode composed of indium tin oxide and having a thickness of 120 nm was formed. After the obtained glass substrate having the transparent electrode was cleaned by irradiation with ultraviolet light and exposure to ozone, the cleaned glass substrate was attached to a vacuum vapor deposition apparatus.

As the first step, N',N"-bis[(4-diphenylamino)phenyl]- N',N"-diphenylbiphenyl-4,4'-diamine was vapor deposited to form a hole injecting layer having a thickness of 60 nm, and N,N,N',N'-tetrakis-(4-biphenyl)-4,4'-benzidine was vapor deposited on the formed layer to form a hole transporting layer having a thickness of 20 mm.

Then, 10,10'-bis[1,1',4',1"]terphenyl-2-yl-9,9'-bianthracenyl as the light emitting material and Compound (5) prepared above as the doping material were simultaneously vapor deposited in amounts such that the ratio of the amounts by weight was 40:2, and a light emitting layer having a thickness of 40 nm was formed.

As the electron injecting layer, tris(8-hydroxyquinolinato)aluminum was vapor deposited to form a layer having a thickness of 10 nm. Lithium fluoride was vapor deposited to form a layer having a thickness of 1 nm, and then aluminum was vapor deposited to form a layer having a thickness of 150 nm. The formed aluminum/lithium fluoride layer worked as the cathode. An organic EL device was prepared as described above.

The prepared device was examined by passing an electric current. Blue light (the maximum wavelength of the emitted light: 476 nm) was emitted at a luminance of light emission of 938 cd/m² under a voltage of 6.9 V and a current density of 10 mA/cm². When the device was examined by continuously passing a direct electric current at an initial luminance of 3,000 cd/m², the half life was 2,000 hours.

### Example 2

An organic EL device was prepared in accordance with the same procedures as those conducted in Example 1 except that Compound (6) prepared above was used in place of Compound (5) used above.

The prepared device was examined by passing an electric current. Blue light (the maximum wavelength of the emitted light: 477 nm) was emitted at a luminance of light emission of 970 cd/m² under a voltage of 6.9 V and a current density of 10 mA/cm². When the device was examined by continuously passing a direct electric current at an initial luminance of 3,000 cd/m², the half life was 2,100 hours.

### Example 3

An organic EL device was prepared in accordance with the same procedures as those conducted in Example 1 except that, in the light emitting layer, 10-(4-(naphthalen-1-yl)phenyl)-9-(naphthalen-3-yl)-anthracene was used as the light emitting material in place of 10,10'-bis[1,1',4',1"]terphenyl-2-yl-9,9'-bianthracenyl, and Compound (15) prepared above was used as the doping material in place of Compound (5) used above.

The prepared device was examined by passing an electric current. Blue light (the maximum wavelength of the emitted light: 472 nm) was emitted at a luminance of light emission of 902 cd/m² under a voltage of 6.8 V and a current density of 10 mA/cm². When the device was examined by continuously passing a direct electric current at an initial luminance of 3,000 cd/m², the half life was 1,900 hours.

### Example 4

An organic EL device was prepared in accordance with the same procedures as those conducted in Example 3 except that Compound (13) prepared above was used in place of Compound (15) used above.

The prepared device was examined by passing an electric current. Blue light (the maximum wavelength of the emitted light: 477 nm) was emitted at a luminance of light emission of 978 cd/m² under a voltage of 6.9 V and a current density of 10 mA/cm². When the device was examined by continuously passing a direct electric current at an initial luminance of 3,000 cd/m², the half life was 3,000 hours.

### Example 5

An organic EL device was prepared in accordance with the same procedures as those conducted in Example 3 except that Compound (30) shown above was used in place of Compound (15) used above.

The prepared device was examined by passing an electric current. Blue light (the maximum wavelength of the emitted light: 469 nm) was emitted at a luminance of light emission of 804 cd/m² under a voltage of 7.0 V and a current density of 10 mA/cm². When the device was examined by continuously passing a direct electric current at an initial luminance of 3,000 cd/m², the half life was 1,800 hours.

### Comparative Example 1

An organic EL device was prepared in accordance with the same procedures as those conducted in Example 1 except that 1,6-bis-(diphenylamino)pyrene was used in place of Compound (5) used above.

The prepared device was examined by passing an electric current. Blue light (the maximum wavelength of the emitted light: 464 nm) was emitted at a luminance of light emission of 615 cd/m² under a voltage of 6.4 V and a current density of 10 mA/cm². When the device was examined by continuously passing a direct electric current at an initial luminance of 3,000 cd/m², the half life was as short as 260 hours.

### Comparative Example 2

An organic EL device was prepared in accordance with the same procedures as those conducted in Example 1 except that 1,6-bis(p,p'-ditolylamino)pyrene was used in place of Compound (5) used above.

The prepared device was examined by passing an electric current. Blue light (the maximum wavelength of the emitted light: 477 nm) was emitted at a luminance of light emission of 976 cd/m² under a voltage of 6.8 V and a current density of 10 mA/cm². When the device was examined by continuously passing a direct electric current at an initial luminance of 3,000 cd/m², the half life was as short as 900 hours.

### Comparative Example 3

An organic EL device was prepared in accordance with the same procedures as those conducted in Example 1 except that 1,4-bis[(2-{4-diphenylamino}phenyl)vinyl]benzene was used in place of Compound (5) used above.

The prepared device was examined by passing an electric current. Blue light (the maximum wavelength of the emitted light: 468 nm) was emitted at a luminance of light emission of 809 cd/m² under a voltage of 6.4 V and a current density of 10 mA/cm². When the device was examined by continuously passing a direct electric current at an initial luminance of 3,000 cd/m², the half life was as short as 1,000 hours.

### INDUSTRIAL APPLICABILITY

The organic EL device using the aromatic amine derivative of the present invention represented by general formula (a) provides a luminance of emitted light sufficient for practical applications under application of a low voltage, exhibits a great efficiency of light emission, suppresses degradation after the use for a long time and has a long life. Therefore, the organic EL device is very useful as the organic EL device having excellent properties for practical applications.

## Claims

1. An aromatic amine derivative represented by following general formula (a): 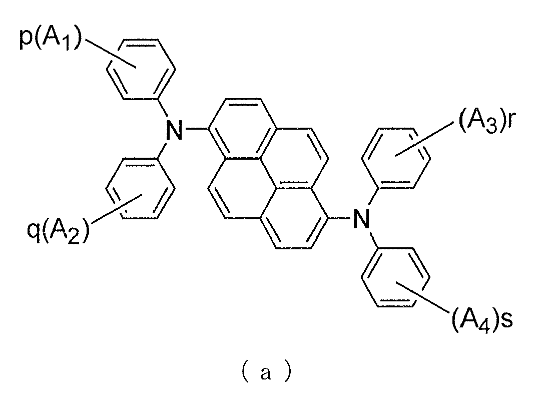 wherein A₁, A₂ and A₃ each independently represent an alkyl group having 1 to 20 carbon atoms, a substituted or unsubstituted aryl group having 5 to 25 carbon atoms, a substituted or unsubstituted aralkyl group having 6 to 25 carbon atoms, a substituted or unsubstituted cycloalkyl group having 3 to 25 carbon atoms, a substituted or unsubstituted alkoxyl group having 1 to 20 carbon atoms, a substituted or unsubstituted aryloxyl group having 5 to 25 carbon atoms, a substituted or unsubstituted arylamino group having 5 to 25 carbon atoms or a substituted or unsubstituted alkylamino group having 1 to 20 carbon atoms;
A₄ represents an alkyl group having 2 to 20 carbon atoms, a substituted or unsubstituted aryl group having 5 to 25 carbon atoms, a substituted or unsubstituted cycloalkyl group having 3 to 25 carbon atoms, a substituted or unsubstituted alkoxyl group having 1 to 20 carbon atoms, a substituted or unsubstituted aryloxyl group having 5 to 25 carbon atoms, a substituted or unsubstituted arylamino group having 5 to 25 carbon atoms or a substituted or unsubstituted alkylamino group having 1 to 20 carbon atoms;
cases in which substituents to any of groups represented by A₁, A₂, A₃ and A₄ are groups having vinyl group are excluded;
p, q, r and s each represent an integer of 0 to 5, and p+q+r+s≧1; when p represents an integer of 2 or greater, a plurality of groups represented by A₁ may be same with or different from each other and may be bonded to each other to form a saturated or unsaturated ring; when q represents an integer of 2 or greater, a plurality of groups represented by A₂ may be same with or different from each other and may be bonded to each other to form a saturated or unsaturated ring; when r represents an integer of 2 or greater, a plurality of groups represented by A₃ may be same with or different from each other and may be bonded to each other to form a saturated or unsaturated ring; and when s represents an integer of 2 or greater, a plurality of groups represented by A₄ may be same with or different from each other and may be bonded to each other to form a saturated or unsaturated ring.

2. An aromatic amine derivative according to Claim 1, wherein at least one of p, q, r and s represents an integer of 2 or greater.

3. An aromatic amine derivative according to any one of Claims 1 and 2, wherein at least one of A₁, A₂, A₃ and A₄ represents a branched alkyl group.

4. An aromatic amine derivative according to Claim 3, wherein the branched alkyl group is isopropyl group or t-butyl group.

5. An aromatic amine derivative according to any one of Claims 1 and 2, wherein at least one of A₁, A₂, A₃ and A₄ represents methyl group.

6. An aromatic amine derivative according to Claim 1, which is one of following Compounds (1), (2), (5), (6), (7), (8), (9), (13), (15), (20), (26), (29), (30) and (33): 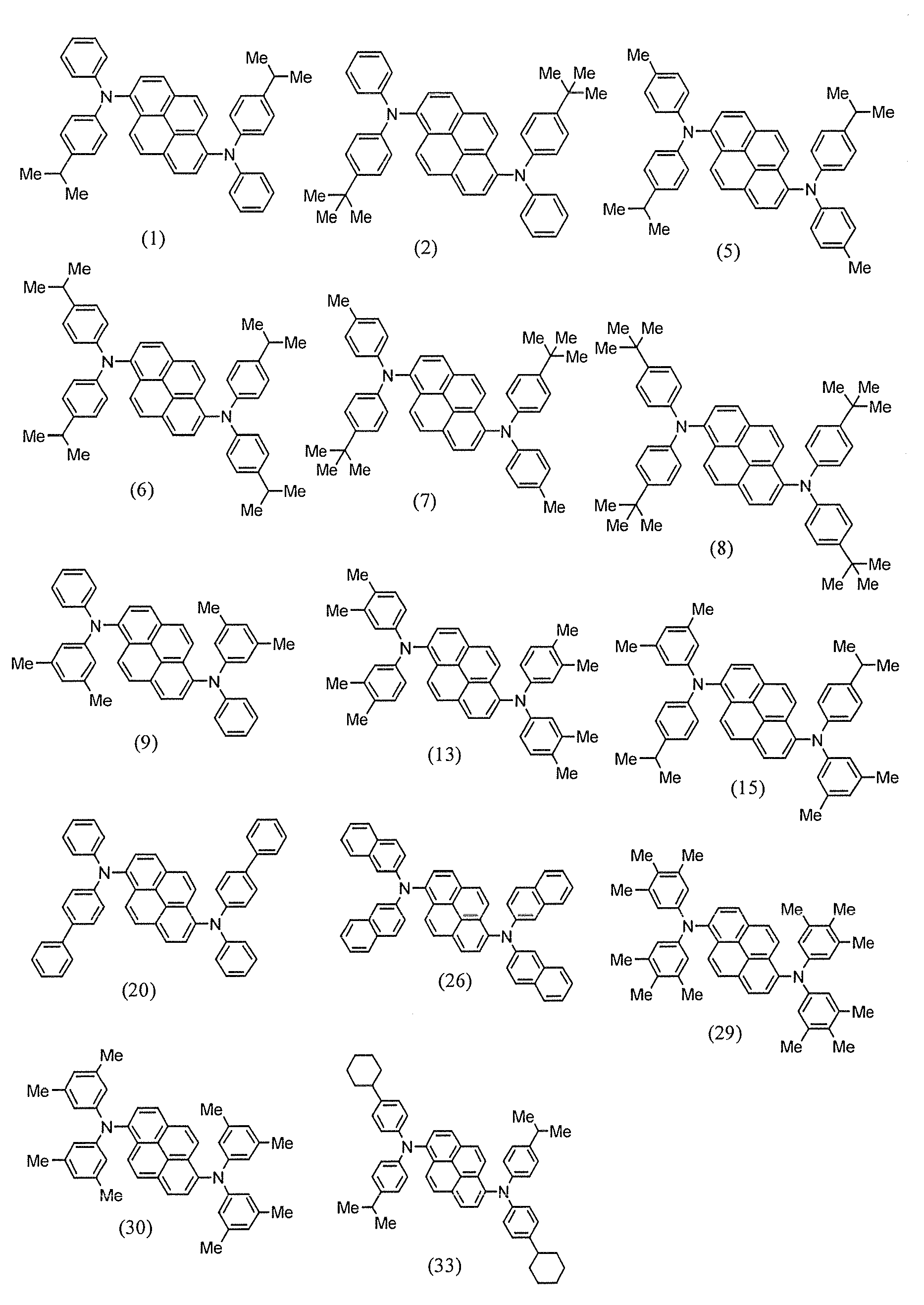 wherein Me represents methyl group.

7. An aromatic amine derivative according to Claim 1, which is a doping material for organic electroluminescence devices.

8. An organic electroluminescence device comprising an anode, a cathode and an organic thin film layer which comprises a single layer or a plurality of layers comprising at least a light emitting layer and is disposed between the anode and the cathode, wherein at least one layer in the organic thin film layer comprises the aromatic amine derivative described in Claim 1 singly or as a component of a mixture.

9. An organic electroluminescence device according to Claim 8, which comprises an organic layer which comprises the aromatic amine derivative as a main component and is disposed between the anode and the light emitting layer.

10. An organic electroluminescence device according to any one of Claims 8 and 9, wherein the light emitting layer comprises 0.1 to 20% by weight of the aromatic amine derivative.
